# EUROPÄISCHE PATENTSCHRIFT

(11) **EP 1 725 229 B1**
(45) Veröffentlichungstag und Bekanntmachung des Hinweises auf die Patenterteilung: **04.03.2009**
(21) Anmeldenummer: 04797701.2
(22) Anmeldetag: 08.11.2004
(51) Int. Cl.: A61K 31/33

(54) **VERWENDUNG VON DESOXYPEGANIN ZUR BEHANDLUNG SCHIZOPHRENER PSYCHOSEN**
USE OF DESOXYPEGANINE FOR THE TREATMENT OF SCHIZOPHRENIC PSYCHOSES
UTILISATION DE DESOXYPEGANINE POUR TRAITER DES PSYCHOSES SCHIZOPHRENES

(30) Priorität: 24.11.2003 DE 10354893
(43) Veröffentlichungstag der Anmeldung: 29.11.2006
(73) Patentinhaber: HF ARZNEIMITTELFORSCHUNG GmbH, 59368 Werne (DE)
(72) Erfinder: MOORMANN, Joachim, 59368 Werne (DE); OPITZ, Klaus, 48157 Münster (DE); WINTERHOFF, Hilke, 48153 Münster (DE)
(74) Vertreter: Flaccus, Rolf-Dieter
(86) Internationale Anmeldenummer: PCT/EP2004/012605
(87) Internationale Veröffentlichungsnummer: WO 2005/053673

(56) Entgegenhaltungen:
- WO-A-03/007966
- DE-C1- 19 906 977
- US-A- 5 633 238
- VOVIN R Y ET AL: "CORRECTION OF AKINETIC-ABULIC SYNDROME IN SCHIZOPHRENICS USING CHOLINOTROPIC DRUGS" ZURNAL NEVROPATALOGII I PSIHIATRII IMENI S.S. KORSAKOVA, IZDATEL'STVO MEDITSINA, MOSCOW, RU, Bd. 91, Nr. 2, Februar 1991 (1991-02), Seiten 111-115, XP001119113 ISSN: 0044-4588

## Beschreibung

Schizophrenie ist eine tief greifende endogene psychiatrische Erkrankung (Psychose), die mit Veränderungen der Gedanken, der Wahrnehmung und des Verhaltens eines Betroffenen einhergeht.

Bei einer schizophrenen Psychose kann praktisch jede psychische Funktion verändert sein. Es zeigen sich eine Vielzahl von Beschwerden, die nicht bei allen Schizophrenie-Erkrankten gleich stark ausgeprägt sein müssen. Grundsätzlich wird bei den Schizophrenien zwischen Grundbeschwerden und akzessorischen Beschwerden unterschieden.

Zu den Grundbeschwerden, bei denen es sich um direkt von der schizophrenen Psychose verursachte Störungen handelt, zählen Denkstörungen, Störungen des Gefühlslebens (Affekt) und des Antriebs, Verlust der Wirklichkeit (Autismus) sowie die so genannte "Ich-Störung", unter der das gespaltene Erleben der eigenen Persönlichkeit verstanden wird.

Zu den akzessorischen Symptomen, d. h. den Beschwerden, die die schizophrenen Patienten in Zusammenhang mit den Grundbeschwerden entwickeln können, gehören Wahnvorstellungen, Halluzinationen, Manerismus und Größenwahn.

Psychotische Patienten verlieren ihre Fähigkeit, geistig und emotional mit anderen Menschen kommunizieren und fortlaufende Ereignisse in ihrem Inhalt und ihrer Bedeutung realistisch beurteilen zu können. Wesentlich ist, dass Schizophrene nicht in einer logischen, Ursache und Wirkung miteinander verknüpfenden Weise denken, die mit Ereignissen in der wirklichen Welt übereinstimmen. So können schizophrene Patienten bizarre Wahnvorstellungen haben, die jegliche Beziehung zur Realität vermissen lassen. Schizophrene erleben auch Halluzinationen, die gewöhnlicherweise akustischer Natur sind.

Neben den angesprochenen Denkstörungen geht die Schizophrenie bei vielen Betroffenen auch mit schweren emotionalen Beeinträchtigungen einher, und sie leiden häufig unter Kontaktarmut und fürchten den Umgang mit anderen Menschen.

Die oben angeführten Schizophreniesymptome sind von emotionalen Störungen, die nicht nur bei Schizophrenie auftreten, abzugrenzen. Zu diesen "nonschizophrenie" Symptomen werden Angst, Spannung, Erregtheit, Schuldgefühle, Depression, Desorientierung und psychosomatische Symptome gezählt.

Die Arten der Beschwerden bei Schizophrenie und bei anderen emotionalen Störungen sind einander sehr ähnlich und können oft nicht unterschieden werden. Daher wurde ein Katalog mit spezifischen Richtlinien für die Diagnose von Schizophrenie entwickelt, der auf dem Fehlen oder Auftreten konkreter, leicht beobachtbarer Verhaltensweisen basiert. So ist für die Diagnose einer Schizophrenie gemäß Leitlinien der Deutschen Gesellschaft für Psychiatrie, ,Psychotherapie und Nervenheilkunde (DGPPN) mindestens ein eindeutiges Symptom aus der Gruppe von Symptomen notwendig, die aus
1. Gedankenlautwerden, Gedankeneingebung oder Gedankenentzug, Gedankenausbreitung;
2. Kontrollwahn, Beeinflussungswalzn, Gefühl des Gemachten bzgl. Körperbewegungen, Gedanken, Tätigkeiten oder Empfindungen; Wahnwahmehmungen ;
3. Kommentierende oder dialogische Stimmen; und
4. Anhaltender, kulturell unangemessener und völlig unrealistischer Wahn
   besteht, oder es müssen mindestens zwei Symptome aus der Gruppe vorliegen, die aus
5. anhaltenden Halluzination jeder Sinnesmodalität;
6. Gedankenabreißen oder Einschiebungen in den Gedankenfluss;
7. Katatone Symptome wie Erregung, Halterungsstereotypien; Negativismus oder Stupor; und
8. "negative" Symptome wie auffällige Apathie, Sprachverarmung, verflachte oder inadäquate Affekte
besteht. Dabei ist zu berücksichtigen, dass die Ursachen der schizophrenen Symptome auch von anderen Entstehungsmöglichkeiten wie etwa Drogen- und Medikamentenmissbrauch, Hirntumore und anderen neurologische Erkrankungen abzugrenzen sind.

Schätzungsweise leidet 1 % der Weltbevölkerung an einer klassischen Schizophrenie, d. h. einer Form dieser Psychose, bei der die Symptome so massiv und eindeutig sind, dass es keine diagnostischen Zweifel gibt.

Die genauen Ursachen einer schizophrenen Erkrankung sind noch unbekannt, jedoch spielen die chemischen Botenstoffe, welche die Nervensignale weiterleiten (Neurotransmitter) eine entscheidende Rolle. Früher nahm man an, dass die Schizophrenie eine Folge der Überproduktion am Neurotransmitter Dopamin sei. Spätere Untersuchungen wiesen jedoch darauf hin, dass ein Teil der Signaltransduktionswege des Dopamins überaktiv ist. So wurde nachgewiesen, dass die zur Behandlung der Schizophrenie eingesetzten Neuroleptika die Wirkung des Dopamins im Gehirn durch Bindung an postsynaptische Dopaminrezeptoren aufheben.

In der bei weitem überwiegenden Zahl von entsprechenden Untersuchungen wurde in Übereinstimmung mit der Hypothese eines überaktiven Dopamin-Signaltransduktionsweges eine verringerte oder unveränderte Monoaminoxidaseaktivität bei Schizophrenie-Patienten gegenüber nicht schizophrenen Probanden festgestellt. Im Gegensatz zu den meisten Untersuchungen konnten Lewine und Meltzer eine signifikante positive Korrelation zwischen den negativen Symptomen und der Aktivität der Monoaminoxidase aus den Blutplättchen männlicher, nicht behandelter Schizophrener nachweisen (Lewine, R.J. und Meltzer, H.Y., Psychiatry Res. 12, 99-109 (1984)). Auch Schildkraut und Mitarbeiter fanden eine erhöhte Monoaminoxidase-Aktivität in den Blutplättchen von Patienten, die unter schizophreniebezogener Depression litten (Schildkraut et al., Schizophr Bull. 6, 220-225(1980); Schildkraut et al., Am J Psychiatry 135, 110-112(1978)).

Daneben gab es in jüngerer Zeit Hinweise aus epidemiologischen Analysen und Verhaltensstudien, die auch eine Rolle neuronaler nikotinischer Rezeptoren (nAchR) bei der Pathogenese neurologischer Erkrankungen einschließlich der Schizophrenie vermuten lassen. So wurde von einer Verringerung nikotinischer Acetylcholin-Rezeptoren bei Schizophrenen berichtet und insbesondere der Alpha-7-Subtyp der nikotinischen Acetylcholin-Rezeptoren wird als für eine schizophrene Erkrankung relevant angesehen. Diese Beobachtungen führten zu einem Interesse an allosterischen Modulatoren nikotinischer Acetylcholin-Rezeptoren, wie beispielsweise Galanthamin, zur Behandlung neurologischer Erkrankungen, die mit einer veränderten Funktion oder Expression nikotinischer Acetylcholin-Rezeptoren in Zusammenhang stehen könnten (Deutsch, S.I. et al., Life Sciences 73, 2333-2361 (2003)).

Neben der psychotherapeutischen Betreuung bildet die Pharmakotherapie mit Antipsychotika, vorwiegend mit Neuroleptika, die Grundlage der Behandlung von schizophrenen Psychosen. Mit der Gabe von Psychopharmaka können die Symptome einer Schizophrenie gelindert werden. Die Neuroleptika können die Gespanntheit vermindern und den Patienten befähigen, über seinen Wahn hinaus mit anderen Menschen umzugehen, so dass die Prognose bei mehr als 50% der von Schizophrenie Betroffenen günstig ist. Diese könnten sich wieder in das soziale Umfeld eingliedern und auch wieder arbeiten. Die Schizophrenie heilen können die Psychopharmaka jedoch nicht.

Die bestehenden Pharmakotherapien haben zudem die Nachteile, dass erhebliche Nebenwirkungen auftreten. So wiesen die Neuroleptika eine lange Liste von starken Nebenwirkungen wie Bewegungsstörungen, unwillkürliche Muskelzuckungen Dämpfung des Empfindens, Müdigkeit, Antriebslosigkeit und Gewichtszunahme auf, von denen die meisten mit Ausnahme der unwillkürlichen Muskelzuckungen nach Absetzen des Medikaments zwar wieder verschwinden. Jedoch lässt das Auftreten der Nebenwirkungen auch erkennen, dass noch immer ein Bedarf an besseren Pharmakotherapeutika besteht, die weniger Nebenwirkungen aufweisen, deren Nebenwirkungen nicht so gravierend sind oder mit denen die Symptome einer schizophrenen Psychose nicht nur unterdrückt, sondern die Erkrankung sogar geheilt werden kann.

Ziel der vorliegenden Erfindung war es daher, Wirkstoffe für die Entwicklung verbesserter Psychopharmaka zur Behandlung schizophrener Psychosen bereitzustellen.

Erfindungsgemäß wird die Aufgabe durch die Verwendung von Desoxypeganin zur Behandlung der Schizophrenie oder zur Herstellung von Arzneimitteln zur Behandlung der Schizophrenie gelöst.

Desoxypeganin (1,2,3,9-Tetrahydropyrrolo [2,1-b] chinazolin), ein Alkaloid der Summenformel C₁₁H₁₂N₂, kommt in Pflanzen der Familie Zygophyllaceae vor. Die Gewinnung von Desoxypeganin erfolgt vorzugsweise durch Isolierung aus der Steppenraute (*Peganum harmala*) oder durch chemische Synthese. Es ist der pharmazeutischen Wissenschaft aus der Literatur sowie insbesondere durch Patentschriften bekannt.

Die DE-A 199 06 978 bzw. WO 00/48582 beschreibt auf Desoxypeganin basierende Arzneimittel zur Therapie der Drogensucht und Drogenabhängigkeit.

Die DE-A 199 06 979 bzw. WO 00/48445 beschreibt auf Desoxypeganin basierende Arzneimittel zur Therapie der Nikotinabhängigkeit.

Die DE-A 199 06 975 bzw. WO 00/48599 beschreibt die Verwendung von Desoxypeganin zur Therapie der Alzheimer'schen Demenz.

In der DE-A 101 63 667 bzw. WO 03/053445 wird die Verwendung von Desoxypeganin zur Behandlung der klinischen Depression offenbart.

Aufgrund seiner pharmakologischen Eigenschaften wird Desoxypeganin zur Gruppe der reversibel wirkenden Cholinesterasehemmstoffe gezählt. Dass Desoxypeganin nicht nur die Acetylcholinesterase, sondern daneben auch Monoaminoxidasen hemmt, ist aus den angeführten Veröffentlichungen in allgemeiner Hinsicht bekannt. Die Monoaminoxidase hemmende Wirkung des Desoxypeganins wird dabei durchgängig als eine bloß ergänzende Wirkung beschrieben, welche die als hauptsächlich betrachtete Acetylcholinesterase-Hemmwirkung des Desoxypeganins verstärken soll.

Aufgrund seines doppelten Wirkmechanismus soll Desoxypeganin vorzugsweise zur Behandlung oder zur Herstellung eines Arzneimittels zur Behandlung einer schizophrenen Psychose verwendet werden, die mit einer erhöhten Monoaminoxidase-Aktivität und/oder verringerten Funktionalität (verringerte Aktivität oder verringerte Expression) nikotinischer Acetylcholin-Rezeptoren, insbesondere des Alpha 7-Subtyps, in Zusammenhang steht.

Die Verabreichung von Desoxypeganin kann peroral oder parenteral erfolgen. Für die orale Verabreichung können bekannte Darreichungsformen wie Tabletten, Dragees, Kapseln, Pastillen verwendet werden. Daneben kommen auch flüssige oder halbflüssige Darreichungsformen, beispielsweise als Trinklösungen in Betracht, wobei der Wirkstoff als Lösung oder Suspension vorliegt. Als Löse- oder Suspendierungsmittel können Wasser, wässrige Medien oder pharmakologisch unbedenkliche Öle (vegetabilische oder Mineralöle) verwendet werden.

Vorzugsweise sind die Desoxypeganin enthaltenden Arzneimittel als Depot-Arzneimittel formuliert, welche in der Lage sind, diesen Wirkstoff über einen längeren Zeitraum in kontrollierter Weise an den Organismus abzugeben.

Darüber hinaus kann Desoxypeganin nach der Erfindung auch rektal (beispielsweise durch Einführung von Suppositorien), inhalativ (durch Einatmen von Aerosolen mit definierter Konzentration und Größenverteilung der Partikel), transdermal (durch wirkstoffhaltige Pflaster, Einreibelösungen, Gele usw.), transmucosal (im Sinne einer Resorption durch die Mund- und Nasenschleimhaut, wobei der Wirkstoff in der Mundhöhle durch Lösung im Speichel freigesetzt wird, oder durch Sprühlösungen und dergleichen in die Nase eingebracht wird), mittels implantierter Behältnisse (die den Wirkstoff passiv-osmotisch oder gesteuert mittels Minipumpen oder dgl. freisetzen), durch intravenöse, intramuskuläre oder subkutane Injektion und intrazerebroventrikulär verabreicht werden.

In Zusammenhang mit parenteraler Verabreichung können transdermale oder transmucosale Darreichungsformen besonders vorteilhaft für die erfindungsgemäße Verabreichung von Desoxypeganin verwendet werden, insbesondere klebende transdermale therapeutische Systeme (Wirkstoffpflaster), wie sie spezifisch für Desoxypeganin in DE-A 199 06 977 beschrieben sind. Diese ermöglichen es, den Wirkstoff über einen längeren Zeitraum in kontrollierter Weise über die Haut an den zu behandelnden Patienten abzugeben.

Nach der Erfindung kann Desoxypeganin sowohl in Form seiner freien Base als auch als Säureadditionssalz zur Behandlung verwendet werden; als Salze werden Desoxypeganinhydrochlorid und Desoxypeganinhydrobromid bevorzugt. Daneben können auch Salze anderer pharmakologisch akzeptabler Säuren verwendet werden, z. B. Citrat, Tartrat oder Acetat.

In gleicher Weise sind anstelle von Desoxypeganin auch dessen in der Literatur beschriebene Derivate zu verstehen, insoweit diese gleichzeitig Hemmstoffe der Acetylcholinesterase und von Monoaminooxidasen sind. Dazu zählen das in Synthetic Communs. 25(4), 569-572 (1995) beschriebene 7-Bromdesoxypeganin, ebenso die in Drug Des. Disc. 14, 1-14 (1996) beschriebenen 7-Halo-6-hydroxy-5-methoxydesoxypeganine der allgemeinen Formel 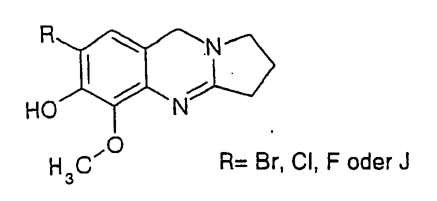 7-Brom-6-hydroxy-5-methoxydesoxypeganin
7-chlor-6-hydroxy-5-methoxydesoxypeganin
7-Fluor-6-hydroxy-5-methoxydesoxypeganin
7-Jod-6-hydroxy-5-methoxydesoxypeganin

Des weiteren sind auch die in Ind. J. Chem. 24B, 789-790 (1985) beschriebenen Derivative des Desoxypeganins verwendbar, nämlich 1,2,3,9-Tetrahydro-6,7-methylenedioxypyrrolo[2,1-b]chinazolin und 2,3-Dihydro-6,7-dimethoxypyrrolo[2,1-b]chinazolin-9(1H)-on.

Die Arzneiformen, welche gemäß vorliegender Erfindung zur Verabreichung von Desoxypeganin verwendet werden, können einen oder mehrere folgender Zusatzstoffe enthalten:
- Antioxydantien, Synergisten, Stabilisatoren;
- Konservierungsmittel;
- Geschmackskorrigentien;
- Färbemittel;
- Lösemittel, Lösungsvermittler;
- Tenside (Emulgatoren, Solubilisatoren, Benetzer, Entschäumer);
- Viskositäts- und Konsistenzbeeinflusser, Gelbildner;
- Resorptionsbeschleuniger;
- Adsorptionsmittel, Feuchthaltemittel, Gleitmittel;
- Zerfalls- und Lösungsbeeinflusser, Füllmittel (Streckmittel), Peptisatoren;
- Freisetzungsverzögerer.
Diese Aufzählung ist nicht abschließend; die in Frage kommenden physiologisch unbedenklichen Substanzen sind dem Fachmann bekannt.

Desoxypeganin wird vorzugsweise in einer Arzneizubereitung verabreicht, welche den Wirkstoff in Anteilen von 0,1 bis 90 Gew.-%, besonders bevorzugt in Anteilen von 2 bis 20 Gew.-%, enthält, jeweils berechnen als freies Desoxypeganin. Die erfindungsgemäß verwendeten desoxypeganinhaltigen, Arzneizubereitungen können darüber hinaus die Zusatzstoffe wie Hilfsstoffe, Trägerstoffe und/oder Stabilisatoren in den dem Fachmann bekannten Mengen enthalten.

Die täglich verabreichte Dosis liegt vorzugsweise im Bereich von 0,1 bis 100 mg, insbesondere von 10 bis 50 mg. Sie ist in Abhängigkeit von den individuellen Voraussetzungen entsprechend einzustellen.

## Patentansprüche

1. Verwendung von Desoxypeganin, als freie Base oder als Säureadditionssalz, oder eines Derivats des Desoxypeganins, das aus der Gruppe ausgewählt ist, die aus 7-Bromdesoxypeganin, 7-Brom-6-hydroxy-5-methoxydesoxypeganin, 7-Chlor-6-hydroxy-5-methoxydesoxypeganin, 7-Fluor-6-hydroxy-5-methoxydesoxypeganin, 7-Jod-6-hydroxy-5-methoxydesoxypeganin, 1,2,3,9-Tetrahydro-6,7-methylenedioxypyrrolo[2,1-b]chinazolin und 2,3-Dihydro-6,7-dimethoxypyrrolo[2,1-b]chinazolin-9(1H)-on besteht, zur Herstellung eines Arzneimittels zur Behandlung einer schizophrenen Psychose, die mit einer erhöhten Monoaminoxidase-Aktivität und/oder verringerten Funktionalität (verringerte Aktivität oder verringerte Expression) nikotinischer Acetylcholin-Rezeptoren in Zusammenhang steht.

2. Verwendung nach Anspruch 1, **dadurch gekennzeichnet, dass** das Arzneimittel den Wirkstoff Desoxypeganin in Anteilen von 0,1 bis 90 Gew.-%, vorzugsweise 2 bis 20 Gew.-%, berechnet als freies Desoxypeganin, enthält.

3. Verwendung nach Anspruch 1 oder 2, **dadurch gekennzeichnet, dass** das Arzneimittel eine Depotwirkung aufweist.

4. Verwendung nach einem der vorangehenden Ansprüche, **dadurch gekennzeichnet, dass** das Arzneimittel ein oral verabreichbares Arzneimittel ist.

5. Verwendung einem der Ansprüche 1 bis 3, **dadurch gekennzeichnet, dass** das Arzneimittel ein parenteral verabreichbares Arzneimittel ist.

6. Verwendung nach Anspruch 5, **dadurch gekennzeichnet, dass** das Arzneimittel ein transdermal verabreichbares Arzneimittel ist.

7. Desoxypeganin, als freie Base oder als Säureadditionssalz, oder eines Derivats des Desoxypeganins, das aus der Gruppe ausgewählt ist, die aus 7-Bromdesoxypeganin, 7-Brom-6-hydroxy-5-methoxydesoxypeganin, 7-Chlor-6-hydroxy-5-methoxydesoxypeganin, 7-Fluor-6-hydroxy-5-methoxydesoxypeganin, 7-Jod-6-hydroxy-5-methoxydesoxypeganin, 1,2,3,9-Tetrahydro-6,7-methylenedioxypyrrolo[2,1-b]chinazolin und 2,3-Dihydro-6,7-dimethoxypyrrolo[2,1-b]chinazolin-9(1H)-on besteht, zur Verwendung zur Behandlung einer schizophrenen Psychose, die mit einer erhöhten Monoaminoxidase-Aktivität und/oder verringerten Funktionalität (verringerte Aktivität oder verringerte Expression) nikotinischer Acetylcholin-Rezeptoren in Zusammenhang steht.

8. Verwendung nach Anspruch 7, **dadurch gekennzeichnet, dass** die verabreichte Dosis im Bereich von 0,1 bis 100 mg, vorzugsweise 10 bis 50 mg, pro Tag liegt.

9. Verwendung nach Anspruch 7 oder 8, **dadurch gekennzeichnet, dass** Desoxypeganin in einer Arzneizubereitung verabreicht wird, welche den Wirkstoff in Anteilen von 0,1 bis 90 Gew.-%, vorzugsweise 2 bis 20 Gew.-%, berechnet als freies Desoxypeganin, enthält.

10. Verwendung nach Anspruch 9, **dadurch gekennzeichnet, dass** Desoxypeganin in einer Arzneizubereitung verabreicht wird, die eine Depotwirkung aufweist.

11. Verwendung nach Anspruch 9 oder 10, **dadurch gekennzeichnet, dass** Desoxypeganin oral verabreicht wird.

12. Verwendung nach Anspruch 9 oder 10, **dadurch gekennzeichnet, dass** Desoxypeganin parenteral verabreicht wird.

13. Verwendung nach Anspruch 12, **dadurch gekennzeichnet, dass** Desoxypeganin transdermal verabreicht wird.

14. Verwendung nach einem der vorangehenden Ansprüche, **dadurch gekennzeichnet, dass** es sich bei den nikotinischen Acetylcholin-Rezeptoren um nikotinische Acetylcholin-Rezeptoren des Alpha 7-Subtyps handelt.

## Claims

1. Use of deoxypeganine, as a free base or as an acid addition salt, or of a derivative of deoxypeganine which is selected from the group consisting of 7-bromodeoxypeganine, 7-bromo-6-hydroxy-5-methoxydeoxypeganine, 7-chloro-6-hydroxy-5-methoxydeoxypeganine, 7-fluoro-6-hydroxy-5-methoxydeoxypeganine, 7-iodo-6-hydroxy-5-methoxydeoxypeganine, 1,2,3,9-tetrahydro-6,7-methylenedioxypyrrolo[2,1-b]quinazoline and 2,3-dihydro-6,7-dimethoxypyrrolo[2,1-b]quinazolin-9 (1H)-one, for producing a medicament for treating a schizophrenic psychosis which is associated with increased monoamine oxidase activity and/or decreased functionality (decreased activity or decreased expression) of nicotinic acetylcholine receptors.

2. Use according to claim 1, **characterized in that** the medicament contains the active substance deoxypeganine in proportions of 0.1 to 90%-wt, preferably 2 to 20%-wt, calculated as free deoxypeganine.

3. Use according to claim 1 or 2, **characterized in that** said medicament has a depot effect.

4. Use according to any one of the preceding claims, **characterized in that** said medicament is a medicament that can be administered orally.

5. Use according to any one of claims 1 to 3, **characterized in that** said medicament is a medicament that can be administered parenterally.

6. Use according to claim 5, **characterized in that** said medicament is a medicament that can be administered transdermally.

7. Deoxypeganine, as a free base or as an acid addition salt, or of a derivative of deoxypeganine which is selected from the group consisting of 7-bromodeoxypeganine, 7-bromo-6-hydroxy-5-methoxydeoxypeganine, 7-chloro-6-hydroxy-5-methoxydeoxypeganine, 7-fluoro-6-hydroxy-5-methoxydeoxypeganine, 7-iodo-6-hydroxy-5-methoxydeoxypeganine, 1,2,3,9-tetrahydro-6,7-methylenedioxypyrrolo[2,1-b]quinazoline and 2,3-dihydro-6,7-dimethoxypyrrolo[2,1-b]quinazolin-9(1H)-one, to be used for the treatment of a schizophrenic psychosis which is associated with increased monoamine oxidase activity and/or decreased functionality (decreased activity or decreased expression) of nicotinic acetylcholine receptors.

8. Use according to claim 7, **characterized in that** the administered daily dose is in the range 0.1 to 100 mg, preferably 10 to 50 mg.

9. Use according to claim 7 or 8, **characterized in that** deoxypeganine is administered in a pharmaceutical preparation containing the active substance in proportions of 0.1 to 90%-wt, preferably 2 to 20%-wt, calculated as free deoxypeganine.

10. Use according to claim 9, **characterized in that** deoxypeganine is administered in a pharmaceutical preparation having depot effect.

11. Use according to claim 9 or 10, **characterized in that** deoxypeganine is administered orally.

12. Use according to claim 9 or 10, **characterized in that** deoxypeganine is administered parenterally.

13. Use according to claim 12, **characterized in that** deoxypeganine is administered transdermally.

14. Use according to any one of the preceding claims, **characterized in that** the said nicotinic acetylcholine receptors are nicotinic acetylcholine receptors of the alpha 7 subtype.

## Revendications

1. Utilisation de la désoxypéganine, sous forme de base libre, ou de sel d'addition d'acide, ou d'un dérivé de la désoxypéganine, qui est choisie dans le groupe formé par la 7-bromo désoxypéganine, la 7-bromo-6-hydroxy-5-méthoxy-désoxypéganine, la 7-chloro-6-hydroxy-5-méthoxy-désoxypéganine, la 7-fluoro-6-hydroxy-5-méthoxy-désoxypéganine, la 7-iodo-6-hydroxy-5-méthoxy-désoxypéganine, la 1,2,3,9-tétrahydro-6,7-méthylène-dioxypyrrolo[2,1-b]-quinazoline et la 2,3-dihydro-6,7-diméthoxypyrrolo[2,1-b]quinazolin-9-(1H)-one, pour préparer un médicament destiné au traitement d'une psychose schizophrène, qui est en rapport avec une activité élevée de monoaminoxydase et/ou une fonctionnalité amoindrie (activité amoindrie ou expression amoindrie) de récepteurs d'acétylcholine nicotiniques.

2. Utilisation selon la revendication 1, **caractérisée en ce que** le médicament contient la substance active, la désoxypéganine, en des proportions de 0,1 à 90% en poids, de préférence de 2 à 20% en poids, calculées en désoxypéganine libre.

3. Utilisation selon la revendication 1 ou 2, **caractérisée en ce que** le médicament présente un effet de dépôt.

4. Utilisation selon l'une quelconque des revendications précédentes, **caractérisée en ce que** le médicament est un médicament pouvant être administré par voie orale.

5. Utilisation selon l'une quelconque des revendications 1 à 3, **caractérisée en ce que** le médicament est un médicament pouvant être administré par voie parentérale.

6. Utilisation selon la revendication 5, **caractérisée en ce que** le médicament est un médicament pouvant être administré par voie transdermique.

7. Désoxypéganine, sous forme de base libre ou de sel d'addition d'acide, ou d'un dérivé de désoxypéganine, qui est choisie dans le groupe formé par la 7-bromo-désoxypéganine, la 7-bromo-6-hydroxy-5-méthoxy-désoxypéganine, la 7-chloro-6-hydroxy-5-méthoxy-désoxypéganine, la 7-fluoro-6-hydroxy-5-méthoxy-désoxypéganine, la 7-iodo-6-hydroxy-5-méthoxy désoxypéganine, la 1,2,3,9-tétrahydro-6,7-méthylène-dioxypyrrolo[2,1-b]-quinazoline et la 2,3-dihydro-6,7-diméthoxypyrrolo[2,1-b]quinazolin-9-(1H)-one, destinée à une utilisation pour le traitement d'une psychose schizophrène, qui est en rapport avec une activité élevée de monoaminoxydase et/ou une fonctionnalité amoindrie (activité amoindrie ou expression amoindrie) de récepteurs d'acétylcholine nicotiniques.

8. Utilisation selon la revendication 7, **caractérisée en ce que** la dose administrée est dans la plage de 0,1 à 100 mg, de préférence de 10 à 50 mg par jour.

9. Utilisation selon la revendication 7 ou 8, **caractérisée en ce que** la désoxypéganine est administrée dans une préparation médicamenteuse, laquelle contient la substance active en des proportions de 0,1 à 90% en poids, de préférence de 2 à 20% en poids, calculées en désoxypéganine libre.

10. Utilisation selon la revendication 9, **caractérisée en ce que** la désoxypéganine est administrée dans une préparation médicamenteuse, qui présente un effet de dépôt.

11. Utilisation selon la revendication 9 ou 10, **caractérisée en ce que** la désoxypéganine est administrée par voie orale.

12. Utilisation selon la revendication 9 ou 10, **caractérisée en ce que** la désoxypéganine est administrée par voie parentérale.

13. Utilisation selon la revendication 12, **caractérisée en ce que** la désoxypéganine est administrée par voie transdermique.

14. Utilisation selon l'une quelconque des revendications précédentes, **caractérisée en ce que**, quant aux récepteurs d'acétylcholine nicotiniques, il s'agit de récepteurs d'acétylcholine nicotiniques du sous-type alpha-7.
